# EUROPEAN PATENT SPECIFICATION

(11) **EP 3 415 178 B1**
(45) Date of publication and mention of the grant of the patent: **11.08.2021**
(21) Application number: 18177285.6
(22) Date of filing: 12.06.2018
(51) Int. Cl.: A61L 31/16, A61B 17/064, A61B 17/068

(54) **SURGICAL STAPLER WITH CONTROLLED HEALING**
CHIRURGISCHES KLAMMERGERÄT MIT KONTROLLIERTER HEILUNG
AGRAFEUSE CHIRURGICALE AVEC RÉGULATION DE LA CICATRISATION

(30) Priority: 13.06.2017 US 201715621572
(43) Date of publication of application: 19.12.2018
(73) Proprietor: Cilag GmbH International, 6300 Zug (CH)
(72) Inventor: WIDENHOUSE, Tamara S., Cincinnati, Ohio 45252 (US); SHELTON, IV, Frederick E., Cincinnati, Ohio 45242 (US)
(74) Representative: Tunstall, Christopher Stephen

(56) References cited:
- EP-A1- 3 135 306
- EP-A1- 3 135 322
- US-A1- 2017 055 986

## Description

### FIELD

Surgical stapling instruments and methods are provided that allow for the healing cascade of stapled tissue to be controlled.

### BACKGROUND

Surgical staplers are used in surgical procedures to close openings in tissue, blood vessels, ducts, shunts, or other objects or body parts involved in the particular procedure. The openings can be naturally occurring, such as passageways in blood vessels or an internal organ like the stomach, or they can be formed by the surgeon during a surgical procedure, such as by puncturing tissue or blood vessels to form a bypass or an anastomosis, or by cutting tissue during a stapling procedure.

Most staplers have a handle with an elongate shaft having a pair of movable opposed jaws formed on an end thereof for holding and forming staples therebetween. The staples are typically contained in a staple cartridge, which can house multiple rows of staples and is often disposed in one of the two jaws for ejection of the staples to the surgical site. In use, the jaws are positioned so that the object to be stapled is disposed between the jaws, and staples are ejected and formed when the jaws are closed and the device is actuated. Some staplers include a knife configured to travel between rows of staples in the staple cartridge to longitudinally cut and/or open the stapled tissue between the stapled rows.

While surgical staplers have improved over the years, a number of problems still present themselves. One common problem is that leaks can occur due to the staple forming holes when penetrating the tissue or other object in which it is disposed. Blood, air, gastrointestinal fluids, and other fluids can seep through the openings formed by the staples, even after the staple is fully formed.

Accordingly, there remains a need for improved devices and methods for stapling tissue, blood vessels, ducts, shunts, or other objects or body parts such that leaking at the site of staple insertion is minimized.

EP 3135306 A1 relates to surgical adjuncts with medicants affected by activator materials. In general, an adjunct is provided with at least one medicant that is configured to be activated by an activator material. The adjunct can be delivered to a tissue of a patient, where the adjunct can be implanted. Various activator materials can be configured to activate the at least one medicant retained by the adjunct, the activation causing any one or more of a variety of actions.

EP 3135322 A1 relates to an adjunct material to provide drug elution in a variety of different temporal and/or spatial patterns. In general, a biocompatible adjunct having a plurality of distinct regions is configured to be delivered to tissue by deployment of staples in a cartridge body of a surgical stapler. Each of the distinct regions can be disposed at a different location on the adjunct material and can have a different adjunct construction. At least two of the regions releasably retain at least one medicant and each of the at least one medicants is releasable from one of the regions in a non-homogeneous manner with respect to at least one of time of release and location of release.

US 2017/055986 A1 relates to one or more adjuncts that can be used in conjunction with surgical instruments. The adjunct(s) can have medicant(s) thereon and/or therein. The medicant(s) can vary depending on the desired effect of the medicant(s) on surrounding tissue. As a non-limiting example, medicant(s) can be provided to influence hemostasis, inflammation, macrophages, and/or fibroblasts. When used in conjunction with a surgical stapler, the adjunct(s) can be disposed between and/or on jaws of the stapler, incorporated into a staple cartridge disposed in the jaws, or otherwise placed in proximity to the staples.

### SUMMARY

The present invention provides a staple cartridge assembly according to claim 1. Embodiments of the invention are defined in the dependent claims. In general, surgical staplers and components thereof are provided that allow a healing cascade of the stapled tissue to be controlled. In particular, surgical staples and components thereof are provided having at least one releasable matrix metalloproteinase (MMP) inhibitor for delivery to tissue surrounding a staple site.

The at least one MMP inhibitor is disposed on at least a portion of the plurality of staples, such as on at least one of a first leg, a second leg (including the distal tips of the first leg and the second leg), and a crown of the plurality of staples. In other aspects, an effective amount of at least one MMP inhibitor can be disposed within and releasable from the adjunct material. The MMP inhibitor can be configured to inhibit one or more of MMP1, MMP2, MMP3, MMP7, MMP8, MMP9, MMP10, MMP11, MMP12, MMP13, MMP14, MMP15, MMP16, MMP17, MMP19, MMP23A, MMP23B, MMP25, MMP25, MMP26, MMP27, and MMP28. The MMP inhibitor can be configured to inhibit a collagenase, a gelatinase, a stromelysin, or a membrane-type MMP. The MMP inhibitor can be a tissue inhibitor of metalloproteinase (TIMP, such as any of TIMP1, TIMP2, TIMP3 or TIMP4). The MMP inhibitor can be one or more of a hydroxymate, carboxylate, carbamoylphosphonate, hydroxyurea, hydrazine, β-lactam, squaric acid, nitrogenous ligand, thiol, and phosphinyl. In some embodiments, the MMP inhibitor can prevent inflammation-causing cells from being attracted to tissue following deployment of the staples.

In one example, an end effector for a surgical instrument is provided that in one implementation has a first jaw having a cartridge body having a plurality of staple cavities configured to seat staples therein and a second jaw having an anvil with a plurality of staple forming cavities formed on a tissue-facing surface thereof. At least one of the first and second jaws can be movable relative to the other. The end effector can also, in some implementations, include a biocompatible adjunct material releasably retained on the cartridge body which is configured to be delivered to tissue by deployment of the staples in the cartridge body. The end effector can include an effective amount of at least one matrix metalloproteinase (MMP) inhibitor which is effective to prevent MMP-mediated extracellular matrix degeneration during wound healing in the tissue in a predetermined manner.

In an unclaimed example, methods are provided for temporarily inhibiting wound healing at a surgical site immediately following surgical stapling of tissue by a surgical stapler. In one embodiment, the method can include engaging tissue between a cartridge assembly and an anvil of the end effector, and actuating the end effector to eject staples from the cartridge assembly into the tissue. The method can optionally include attaching an adjunct material to an end effector of the surgical stapler and extending the staples through the adjunct material to maintain the adjunct material at the surgical site. At least one of a portion of the plurality of staples and an optional adjunct material which can be attached to an end effector of the surgical stapler can include an effective amount of at least one matrix metalloproteinase (MMP) inhibitor. Release of the MMP inhibitor into the tissue surrounding the staple insertion site can prevent MMP-mediated extracellular matrix degeneration during wound healing in the tissue in a predetermined manner.

The at least one MMP inhibitor is disposed on at least a portion of the plurality of staples, such as on at least one of a first leg, a second leg (including the distal tips of the first leg and the second leg), and a crown of the plurality of staples. An effective amount of at least one MMP inhibitor can be disposed within and releasable from the adjunct material. The MMP inhibitor can be configured to inhibit one or more of MMP1, MMP2, MMP3, MMP7, MMP8, MMP9, MMP10, MMP11, MMP12, MMP13, MMP14, MMP15, MMP16, MMP17, MMP19, MMP23A, MMP23B, MMP25, MMP25, MMP26, MMP27, and MMP28. The MMP inhibitor can be configured to inhibit a collagenase, a gelatinase, a stromelysin, or a membrane-type MMP. The MMP inhibitor can be a tissue inhibitor of metalloproteinase (TIMP, such as any of TIMP1, TIMP2, TIMP3 or TIMP4). The MMP inhibitor can be one or more of a hydroxymate, carboxylate, carbamoylphosphonate, hydroxyurea, hydrazine, β-lactam, squaric acid, nitrogenous ligand, thiol, and phosphinyl. In some embodiments, the MMP inhibitor can prevent inflammation-causing cells (for example, at least one of neutrophils, helper T cells, and macrophages) from being attracted to the tissue following deployment of the staples. In some aspects, the tissue can be colon tissue, such as colon tissue immediately following surgical resection.

### BRIEF DESCRIPTION OF DRAWINGS

This invention will be more fully understood from the following detailed description taken in conjunction with the accompanying drawings, in which:
FIG. 1 is a perspective view of one embodiment of a surgical stapler.
FIG. 2 is an exploded view of a distal portion of the surgical stapler of FIG. 1.
FIG. 3 is a perspective view of a firing bar of the surgical stapler of FIG. 1, the firing bar having an E-beam at a distal end thereof.
FIG. 4 is a perspective view of another embodiment of a surgical stapler.
FIG. 5 is a perspective view of yet another embodiment of a surgical stapler.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. Sizes and shapes of the systems and devices, and the components thereof, can depend at least on the anatomy of the subject in which the systems and devices will be used, the size and shape of components with which the systems and devices will be used, and the methods and procedures in which the systems and devices will be used.

It will be appreciated that the terms "proximal" and "distal" are used herein with reference to a user, such as a clinician, gripping a handle of an instrument. Other spatial terms such as "front" and "back" similarly correspond respectively to distal and proximal. It will be further appreciated that for convenience and clarity, spatial terms such as "vertical" and "horizontal" are used herein with respect to the drawings. However, surgical instruments are used in many orientations and positions, and these spatial terms are not intended to be limiting and absolute.

Various exemplary devices and methods are provided for performing surgical procedures. In some embodiments, the devices and methods are provided for open surgical procedures, and in other embodiments, the devices and methods are provided for laparoscopic, endoscopic, and other minimally invasive surgical procedures. The devices may be fired directly by a human user or remotely under the direct control of a robot or similar manipulation tool. However, a person skilled in the art will appreciate that the various methods and devices disclosed herein can be used in numerous surgical procedures and applications. Those skilled in the art will further appreciate that the various instruments disclosed herein can be inserted into a body in any way, such as through a natural orifice, through an incision or puncture hole formed in tissue, or through an access device, such as a trocar cannula. For example, the working portions or end effector portions of the instruments can be inserted directly into a patient's body or can be inserted through an access device that has a working channel through which the end effector and elongated shaft of a surgical instrument can be advanced.

During performance of a surgical procedure, tissue of a patient can be wounded (e.g., cut, torn, punctured, etc.) in any of a variety of ways. The wounding may be an intended aspect of the surgical procedure, such as in an anastomosis procedure and/or when tissue is cut and fastened using a surgical device such as a surgical stapler. The wounded tissue typically heals over time in generally the same way for all patients.

Wound healing is traditionally considered to include four stages: hemostasis, inflammation, proliferation, and remodeling. The hemostasis stage generally involves blood clotting, *e.g*., stopping bleeding. In general, damaged blood vessels constrict to slow blood flow, platelets aggregate to help seal the wound site, the platelets activate fibrin to further facilitate wound sealing, and a blood clot forms at the wound site. The inflammation stage generally involves cleaning of the wound site. In general, the immune system provides a response to the threat of possible infection at the wound site via signaling to defensive immune cells such as neutrophils and macrophages. The proliferation stage generally involves rebuilding tissue with tissue growth and angiogenesis (blood vessel growth). In general, fibroblasts arrive at the wound site, lay down collagen, release growth factors that attract epithelial cells, and the epithelial cells attract endothelial cells. The remodeling stage, also referred to as a maturation stage, generally involves strengthening scar tissue at the wound site. In general, collagen fibers align and crosslink, and the scar matures to eventually fade away.

While each of wound healing's four stages involves a different aspect of the healing process, stages typically overlap with one another. Namely, each of the last three stages typically overlaps with its preceding stage, *e.g*., inflammation overlaps with hemostasis, proliferation overlaps with inflammation, and remodeling overlaps with proliferation. The speed at which the transition between stages occurs generally affects the speed of overall wound healing and thus generally affects patient recovery time, chances of complications arising, and/or patient comfort. Similarly, the length of each of the four individual stages generally affects the speed of overall wound healing and the patient's general recovery.

Matrix metalloproteinases (MMPs) are a family of proteases that breakdown components of the extracellular matrix (ECM) of tissue under a variety of physiological and pathological conditions, including during wound healing. These enzymes remove dead and devitalized tissue, help to remodel the underlying connective tissue of the ECM, promote inflammatory cell migration into the wound site, and assist in angiogenesis.

During the inflammation stage of wound healing, MMPs break down damaged ECM located at the edges of wounds. This enables new ECM molecules (such as, for example, collagen, elastin, and fibronectin) synthesized by cells located at or attracted to the wound site during the later stages of wound healing to eventually merge into and become part of the intact ECM, thereby resulting in wound closure and healing. Further, expression of certain MMPs (for example, MMP1, MMP9, and MMP8) immediately following wounding have been associated with the recruitment of pro-inflammatory immune cells to the site of wound formation (such as, but not limited to, neutrophils, helper T cells, and macrophages) as well as expression of pro-inflammatory substances and proteins.

As discussed in more detail in the Examples section, immediately following tissue stapling, cells present at the site of staple insertion release MMPs, which begin the process of degrading the ECM (in particular, the collagen components of the ECM) at and near the wound caused by staple insertion in order to facilitate the initial stages of wound healing. However, without being bound to theory and as illustrated in the Examples, it is believed that this natural process can, at least initially (*i.e.* up to about three days following staple insertion), result in the weakening of tissue surrounding the staple site (due, for example, to collagen degeneration in the ECM), making it susceptible to tearing and other complications (such as, leaking of blood, air, and other fluids through the openings formed by the staples as well as, *e.g.,* anastomotic leakage following bowel resection). As such, and again without being bound to theory, it is believed that delivering substances capable of inhibiting MMPs to wound sites in tissue (for example, intestinal tissue) immediately after staple insertion can prevent or minimize the ECM degeneration associated with the initial stages of wound healing, thereby strengthening the staple insertion site and making it less likely to leak or rupture.

Thus, it can be desirable to use one or more MMP inhibitors, in conjunction with surgical instruments, such as surgical staplers, to help improve surgical procedures. MMP inhibitors are molecules capable of inhibiting or decreasing the proteolytic activity of MMPs on ECM components at a wound site. Additionally, MMP inhibition immediately following wound formation can prevent additional inflammation-promoting immune cells from being attracted to the wound site, which can lead to further structural weakening of the ECM. Without being bound to theory, by preventing the natural MMP-mediated ECM degradation that occurs immediately following staple insertion, MMP inhibitors may be able to prevent the tissue leakage complications associated with tissue stapling by strengthening the tissue surrounding the wound site. In some instances, the MMP inhibitors delivered to sites of tissue stapling as provided by the devices and in accordance with the methods provided herein may increase the incidence of scar tissue formation, leading to the minimization of tissue movement in and around the staple puncture sites that can occur from tissue deformation that occurs after stapling (e.g., lung inflation, gastrointestinal tract distension, etc.). It will be recognized by one skilled in the art that a staple puncture site may serve as a stress concentration and that the size of the hole created by the staple will grow when the tissue around it is placed under tension. Accordingly, restricting tissue movement around these puncture sites by preventing MMP-mediated degradation of the ECM (for example, MMP-mediated proteolysis of collagen) can encourage scar tissue formation and thereby minimize the size the holes may grow to under tension, as well as the potential for leakage.

Further clinical studies directed to MMPs and use of MMP inhibitors in wound healing can be found in Argen et al., Surgery, 2006, 140(1):72-82; Krarup, et al., Int J Colorectal Dis, 2013, 28:1151-1159; Bosmans et al., BMC Gastroenterology (2015) 15:180; Holte et al., Brit. J. Surg., 2009, 96:650-54; Siemonsma et al., Surgery, 2002, 133(3):268-276; Klein et al, Eur. Surg. Res., 2011, 46:26-31; Moran et al., World J. Emergency Surgery, 2007, 2:13; Kaemmer et al., J. Surg. Res., 2010,163, e67-e72; Martens et al., Gut, 1991, 32, 1482-87; Fatouros et al., 1999, Eur. J. Surg., 165(10):986-92; Savage et al., 1997, 40(8): 962-70; Oines et al., World J Gastroenterol, 2014 20(35): 12637-48; Kiyama et al., Wound Repair and Regen., 10(5):308-13; Raptis et al., Int. J. Colorectal Dis., 2011; de Hingh et al., Int. J. Colorectal. Dis., 2002, 17:348-54; and Hayden et al., 2011, J. Surgical Res., 168:315-324.

### SURGICAL STAPLING INSTRUMENTS

A variety of surgical instruments can be used in conjunction with the medicant(s) disclosed herein. The surgical instruments can include surgical staplers. A variety of surgical staplers can be used, for example linear surgical staplers and circular staplers. In general, a linear stapler can be configured to create longitudinal staple lines and can include elongate jaws with a cartridge coupled thereto containing longitudinal staple rows. The elongate jaws can include a knife or other cutting element capable of creating a cut between the staple rows along tissue held within the jaws. In general, a circular stapler can be configured to create annular staple lines and can include circular jaws with a cartridge containing annular staple rows. The circular jaws can include a knife or other cutting element capable of creating a cut inside of the rows of staples to define an opening through tissue held within the jaws. The staplers can be used in a variety of different surgical procedures on a variety of tissues in a variety of different surgical procedures, for example in thoracic surgery or in gastric surgery.

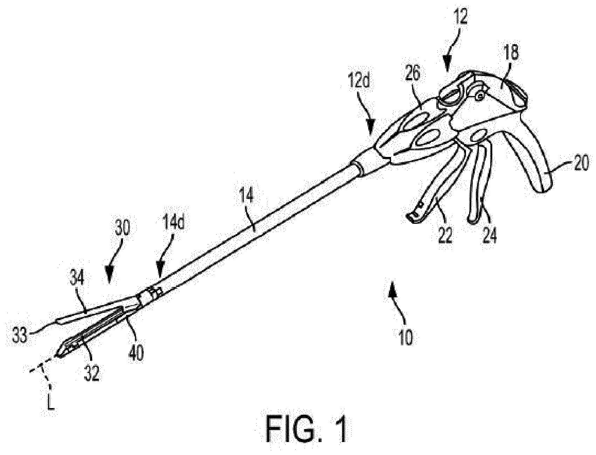

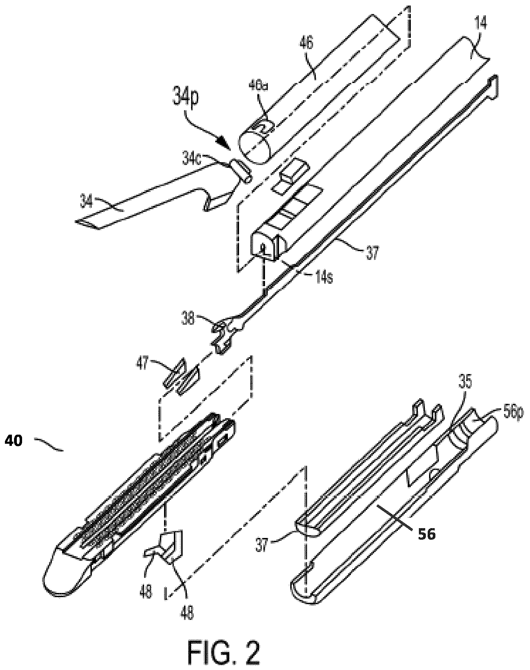

FIG. **1** illustrates one example of a linear surgical stapler 10 suitable for use with one or more adjunct(s) and/or medicant(s). The stapler 10 generally includes a handle assembly 12, a shaft 14 extending distally from a distal end 12d of the handle assembly 12, and an end effector 30 at a distal end 14d of the shaft 14. The end effector 30 has opposed lower and upper jaws 32, 34, although other types of end effectors can be used with the shaft 14, handle assembly 12, and components associated with the same. The lower jaw 32 has a staple channel 56 configured to support a staple cartridge 40, and the upper jaw 34 has an anvil surface 33 that faces the lower jaw 32 and that is configured to operate as an anvil to help deploy staples of the staple cartridge 40 (the staples are obscured in FIG. **1** and FIG. **2****).** At least one of the opposed lower and upper jaws 32, 34 is moveable relative to the other lower and upper jaws 32, 34 to clamp tissue and/or other objects disposed therebetween. In some implementations, one of the opposed lower and upper jaws 32, 34 may be fixed or otherwise immovable. In some implementations, both of the opposed lower and upper jaws 32, 34 may be movable. Components of a firing system can be configured to pass through at least a portion of the end effector 30 to eject the staples into the clamped tissue. In various implementations a knife blade 36 or other cutting element can be associated with the firing system to cut tissue during the stapling procedure.

Operation of the end effector 30 can begin with input from a user, *e.g.,* a clinician, a surgeon, etc., at the handle assembly 12. The handle assembly 12 can have many different configurations designed to manipulate and operate the end effector 30 associated therewith. In the illustrated example, the handle assembly 12 has a pistol-grip type housing 18 with a variety of mechanical and/or electrical components disposed therein to operate various features of the instrument 10. For example, the handle assembly 12 can include a rotation knob 26 mounted adjacent a distal end 12d thereof which can facilitate rotation of the shaft 14 and/or the end effector 30 with respect to the handle assembly 12 about a longitudinal axis L of the shaft 14. The handle assembly 12 can further include clamping components as part of a clamping system actuated by a clamping trigger 22 and firing components as part of the firing system that are actuated by a firing trigger 24. The clamping and firing triggers 22, 24 can be biased to an open position with respect to a stationary handle 20, for instance by a torsion spring. Movement of the clamping trigger 22 toward the stationary handle 20 can actuate the clamping system, described below, which can cause the jaws 32, 34 to collapse towards each other and to thereby clamp tissue therebetween. Movement of the firing trigger 24 can actuate the firing system, described below, which can cause the ejection of staples from the staple cartridge 40 disposed therein and/or the advancement the knife blade 36 to sever tissue captured between the jaws 32, 34. A person skilled in the art will recognize that various configurations of components for a firing system, mechanical, hydraulic, pneumatic, electromechanical, robotic, or otherwise, can be used to eject staples and/or cut tissue.

As shown in FIG. **2****,** the end effector 30 of the illustrated implementation has the lower jaw 32 that serves as a cartridge assembly or carrier and the opposed upper jaw 34 that serves as an anvil. The staple cartridge 40, having a plurality of staples therein, is supported in a staple tray 37, which in turn is supported within a cartridge channel of the lower jaw 32. The upper jaw 34 has a plurality of staple forming pockets (not shown), each of which is positioned above a corresponding staple from the plurality of staples contained within the staple cartridge 40. The upper jaw 34 can be connected to the lower jaw 32 in a variety of ways, although in the illustrated implementation the upper jaw 34 has a proximal pivoting end 34p that is pivotally received within a proximal end 56p of the staple channel 56, just distal to its engagement to the shaft 14. When the upper jaw 34 is pivoted downwardly, the upper jaw 34 moves the anvil surface 33 and the staple forming pockets formed thereon move toward the opposing staple cartridge 40.

Various clamping components can be used to effect opening and closing of the jaws 32, 34 to selectively clamp tissue therebetween. As illustrated, the pivoting end 34p of the upper jaw 34 includes a closure feature 34c distal to its pivotal attachment with the staple channel 56. Thus, a closure tube 46, whose distal end includes a horseshoe aperture 46a that engages the closure feature 34c, selectively imparts an opening motion to the upper jaw 34 during proximal longitudinal motion and a closing motion to the upper jaw 34 during distal longitudinal motion of the closure tube 46 in response to the clamping trigger 22. As mentioned above, in various implementations, the opening and closure of the end effector 30 may be effected by relative motion of the lower jaw 32 with respect to the upper jaw 34, relative motion of the upper jaw 34 with respect to the lower jaw 32, or by motion of both jaws 32, 34 with respect to one another.

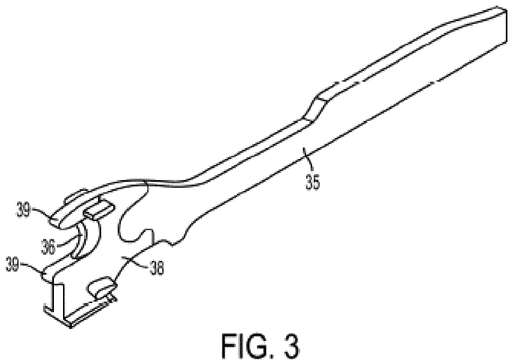

The firing components of the illustrated implementation includes a firing bar 35, as shown in FIG. 3, having an E-beam 38 on a distal end thereof. The firing bar 35 is encompassed within the shaft 14, for example in a longitudinal firing bar slot 14s of the shaft 14, and guided by a firing motion from the handle 12. Actuation of the firing trigger 24 can affect distal motion of the E-beam 38 through at least a portion of the end effector 30 to thereby cause the firing of staples contained within the staple cartridge 40. As illustrated, guides 39 projecting from a distal end of the E-Beam 38 can engage a wedge sled 47 shown in FIG. 2, which in turn can push staple drivers 48 upwardly through staple cavities 41 formed in the staple cartridge 40. Upward movement of the staple drivers 48 applies an upward force on each of the plurality of staples within the cartridge 40 to thereby push the staples upwardly against the anvil surface 33 of the upper jaw 34 and create formed staples.

In addition to causing the firing of staples, the E-beam 38 can be configured to facilitate closure of the jaws 32, 34, spacing of the upper jaw 34 from the staple cartridge 40, and/or severing of tissue captured between the jaws 32, 34. In particular, a pair of top pins and a pair of bottom pins can engage one or both of the upper and lower jaws 32, 34 to compress the jaws 32, 34 toward one another as the firing bar 35 advances through the end effector 30. Simultaneously, the knife 36 extending between the top and bottom pins can be configured to sever tissue captured between the jaws 32, 34.

In use, the surgical stapler 10 can be disposed in a cannula or port and disposed at a surgical site. A tissue to be cut and stapled can be placed between the jaws 32, 34 of the surgical stapler 10. Features of the stapler 10 can be maneuvered as desired by the user to achieve a desired location of the jaws 32,34 at the surgical site and the tissue with respect to the jaws 32, 34. After appropriate positioning has been achieved, the clamping trigger 22 can be pulled toward the stationary handle 20 to actuate the clamping system. The trigger 22 can cause components of the clamping system to operate such that the closure tube 46 advances distally through at least a portion of the shaft 14 to cause at least one of the jaws 32, 34 to collapse towards the other to clamp the tissue disposed therebetween. Thereafter, the trigger 24 can be pulled toward the stationary handle 20 to cause components of the firing system to operate such that the firing bar 35 and/or the E-beam 38 are advanced distally through at least a portion of the end effector 30 to effect the firing of staples and optionally to sever the tissue captured between the jaws 32, 34.

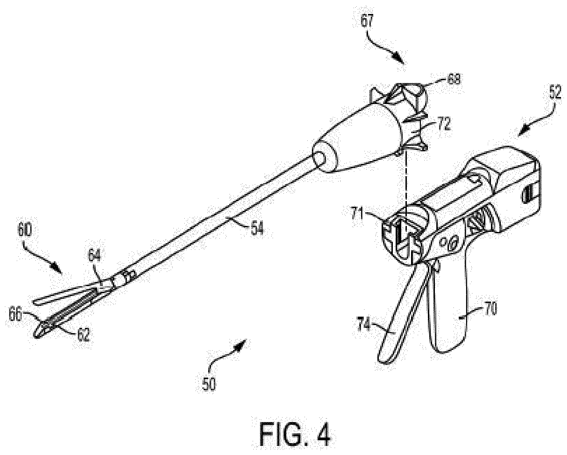

Another example of a surgical instrument in the form of a linear surgical stapler 50 is illustrated in FIG. **4****.** The stapler 50 can generally be configured and used similar to the stapler 10 of FIG. **1****.** Similar to the surgical instrument 10 of FIG. **1****,** the surgical instrument 50 includes a handle assembly 52 with a shaft 54 extending distally therefrom and having an end effector 60 on a distal end thereof for treating tissue. Upper and lower jaws 64, 62 of the end effector 60 can be configured to capture tissue therebetween, staple the tissue by firing of staples from a cartridge 66 disposed in the lower jaw 62, and/or to create an incision in the tissue. In this implementation, an attachment portion 67 on a proximal end of the shaft 54 can be configured to allow for removable attachment of the shaft 54 and the end effector 60 to the handle assembly 52. In particular, mating features 68 of the attachment portion 67 can mate to complementary mating features 71 of the handle assembly 52. The mating features 68, 71 can be configured to couple together via, *e.g.,* a snap fit coupling, a bayonet type coupling, etc., although any number of complementary mating features and any type of coupling can be used to removably couple the shaft 54 to the handle assembly 52. Although the entire shaft 54 of the illustrated implementation is configured to be detachable from the handle assembly 52, in some implementations, the attachment portion 67 can be configured to allow for detachment of only a distal portion of the shaft 54. Detachable coupling of the shaft 54 and/or the end effector 60 can allow for selective attachment of a desired end effector 60 for a particular procedure, and/or for reuse of the handle assembly 52 for multiple different procedures.

The handle assembly 52 can have one or more features thereon to manipulate and operate the end effector 60. By way of non-limiting example, a rotation knob 72 mounted on a distal end of the handle assembly 52 can facilitate rotation of the shaft 54 and/or the end effector 60 with respect to the handle assembly 52. The handle assembly 52 can include clamping components as part of a clamping system actuated by a movable trigger 74 and firing components as part of a firing system that can also be actuated by the trigger 74. Thus, in some implementations, movement of the trigger 74 toward a stationary handle 70 through a first range of motion can actuate clamping components to cause the opposed jaws 62, 64 to approximate toward one another to a closed position. In some implementations, only one of the opposed jaws 62, 24 can move to the jaws 62, 64 to the closed position. Further movement of the trigger 74 toward the stationary handle 70 through a second range of motion can actuate firing components to cause the ejection of the staples from the staple cartridge 66 and/or the advancement of a knife or other cutting element (not shown) to sever tissue captured between the jaws 62, 64.

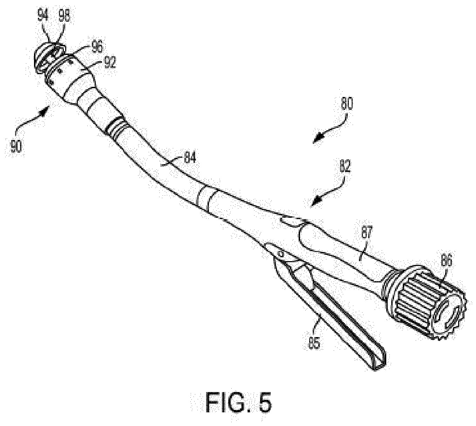

One example of a surgical instrument in the form of a circular surgical stapler 80 is illustrated in FIG. 5. The stapler 80 can generally be configured and used similar to the linear staplers 10, 50 of FIG. 1 and FIG. 4, but with some features accommodating its functionality as a circular stapler. Similar to the surgical instruments 10, 50, the surgical instrument 80 includes a handle assembly 82 with a shaft 84 extending distally therefrom and having an end effector 90 on a distal end thereof for treating tissue. The end effector 90 can include a cartridge assembly 92 and an anvil 94, each having a tissue-contacting surface that is substantially circular in shape. The cartridge assembly 92 and the anvil 94 can be coupled together via a shaft 98 extending from the anvil 94 to the handle assembly 82 of the stapler 80, and manipulating an actuator 85 on the handle assembly 82 can retract and advance the shaft 98 to move the anvil 94 relative to the cartridge assembly 92. The anvil 94 and cartridge assembly 92 can perform various functions and can be configured to capture tissue therebetween, staple the tissue by firing of staples from a cartridge 96 of the cartridge assembly 92 and/or can create an incision in the tissue. In general, the cartridge assembly 92 can house a cartridge containing the staples and can deploy staples against the anvil 94 to form a circular pattern of staples, *e.g.,* staple around a circumference of a tubular body organ.

In one implementation, the shaft 98 can be formed of first and second portions (not shown) configured to releasably couple together to allow the anvil 94 to be detached from the cartridge assembly 92, which may allow greater flexibility in positioning the anvil 94 and the cartridge assembly 92 in a body of a patient. For example, the first portion of the shaft can be disposed within the cartridge assembly 92 and extend distally outside of the cartridge assembly 92, terminating in a distal mating feature. The second portion of the shaft 84 can be disposed within the anvil 94 and extend proximally outside of the cartridge assembly 92, terminating in a proximal mating feature. In use, the proximal and distal mating features can be coupled together to allow the anvil 94 and cartridge assembly 92 to move relative to one another.

The handle assembly 82 of the stapler 80 can have various actuators disposed thereon that can control movement of the stapler. For example, the handle assembly 82 can have a rotation knob 86 disposed thereon to facilitate positioning of the end effector 90 via rotation, and/or the trigger 85 for actuation of the end effector 90. Movement of the trigger 85 toward a stationary handle 87 through a first range of motion can actuate components of a clamping system to approximate the jaws, i.e. move the anvil 94 toward the cartridge assembly 92. Movement of the trigger 85 toward the stationary handle 87 through a second range of motion can actuate components of a firing system to cause the staples to deploy from the staple cartridge assembly 92 and/or cause advancement of a knife to sever tissue captured between the cartridge assembly 92 and the anvil 94.

The illustrated examples of surgical stapling instruments 10, 50, and 80 provide only a few examples of many different configurations, and associated methods of use, that can be used in conjunction with the disclosures provided herein. Although the illustrated examples are all configured for use in minimally invasive procedures, it will be appreciated that instruments configured for use in open surgical procedures, *e.g*., open linear staplers as described in U.S. Pat. No. 8,317,070 entitled "Surgical Stapling Devices That Produce Formed Staples Having Different Lengths" and filed February 28, 2007, can be used in conjunction with the disclosures provided herein. Greater detail on the illustrated examples, as well as additional examples of surgical staplers, components thereof, and their related methods of use, are provided in U.S. Pat. Pub. No. 2013/0256377 entitled "Layer Comprising Deployable Attachment Members" and filed February 8, 2013, U.S. Pat. No. 8,393,514 entitled "Selectively Orientable Implantable Fastener Cartridge" and filed September 30, 2010, U.S. Pat. No. 8,317,070 entitled "Surgical Stapling Devices That Produce Formed Staples Having Different Lengths" and filed February 28, 2007, U.S. Pat. No. 7,143,925 entitled "Surgical Instrument Incorporating EAP Blocking Lockout Mechanism" and filed June 21, 2005, U.S. Pat. Pub. No. 2015/0134077 entitled "Sealing Materials for Use in Surgical Stapling" and filed November 8, 2013, entitled "Sealing Materials for Use in Surgical Procedures, and filed on November 8, 2013, U.S. Pat. Pub. No. 2015/0134076, entitled "Hybrid Adjunct Materials for Use in Surgical Stapling," and filed on November 8, 2013, U.S. Pat. Pub. No. 2015/0133996, entitled "Positively Charged Implantable Materials and Method of Forming the Same," and filed on November 8, 2013, U.S. Pat. Pub. No. 2015/0129634, entitled "Tissue Ingrowth Materials and Method of Using the Same," and filed on November 8, 2013, U.S. Pat. Pub. No. 2015/0133995, entitled "Hybrid Adjunct Materials for Use in Surgical Stapling," and filed on November 8, 2013, U.S. Pat. App. No. 14/226,142, entitled "Surgical Instrument Comprising a Sensor System," and filed on March 26, 2014, and U.S. Pat. App. No. 14/300,954, entitled "Adjunct Materials and Methods of Using Same in Surgical Methods for Tissue Sealing," and filed on June 10, 2014.

### MMP INHIBITORS

MMPs, which comprise a family of more than 20 members, use Zn²⁺ in their active sites to catalyze hydrolyses of ECM components such as collagen. Based on their substrate specificities, they can be broadly classified into three subfamilies: collagenases, stromelysins and gelatinases. Proteins of the MMP family are involved in the breakdown of extracellular matrix in normal physiological processes, such as embryonic development, reproduction, angiogenesis, bone development, wound healing, cell migration, learning and memory, as well as in pathological processes, such as arthritis, intracerebral hemorrhage, and metastasis. Most MMPs are secreted as inactive proproteins, which are activated when cleaved by extracellular proteinases. The enzyme encoded by this gene degrades type IV and V collagens and other extracellular matrix proteins.

Under normal physiological conditions, MMPs function in wound healing and tissue remodeling. However, when these enzymes are over activated, they can over-degrade ECM, resulting in disease conditions. For example, MMP-2 and MMP-9 (both are gelatinases) are thought to be involved in the pathogenesis of inflammatory, infectious, and neoplastic diseases in many organs. Excess activity of MMP-8, also known as collagenase-2 or neutrophil collagenase, is associated with diseases such as pulmonary emphysema and osteoarthritis. *See* Balbin et al., "Collagenase 2 (MMP-8) expression in murine tissue-remodeling processes, analysis of its potential role in postpartum involution of the uterus," J. Biol. Chem., 273(37): 23959-23968 (1998). Excess activity of MMP-12, also known as macrophage elastase or metalloelastase, plays a key role in tumor invasion, arthritis, atherosclerosis, Alport syndrome, and chronic obstructive pulmonary disease (COPD). MMP-1 and MMP-13 are involved in the proteolysis of collagen. Excessive degradation of collagen is associated with the development of various diseases, including osteoarthritis. *See e.g.,* P. G. Mitchell et al., "Cloning, expression, and type II collagenolytic activity of matrix metalloproteinase-13 from human osteoarthritic cartilage," J Clin invest. 1996 Feb. 1; 97(3): 761-768.

A "matrix metalloproteinase inhibitor" or "MMP inhibitor," as used herein, is any chemical compound that inhibits by at least five percent the proteolytic activity (such as inhibits any of 5%, 10%, 15%, 20%, 25%, 30%, 35%, 40%, 45%, 50%, 55%, 60%, 65%, 70%, 75%, 80%, 85%, 90%, 95%, or 100% of the proteolytic activity) of at least one matrix metalloproteinase enzyme that is naturally occurring in a mammal (such as an MMP that naturally is expressed during wound healing). Many MMP inhibitors are known in the art. For example, existing MMP inhibitors can be based on hydroxamic acid derivatives, suflonyl amino acid, and sulfonylamino hydroxamic acid derivatives. The hydroxamic acid moiety in these inhibitors binds to the MMP active site Zn²⁺ to inhibit enzymatic activities. Further, numerous peptides are known matrix metalloproteinase inhibitors.

Non-limiting specific examples of matrix metalloproteinase inhibitors that inhibit or decrease the proteolytic activity of MMPs include, without limitation, of exogenous MMP inhibitors, Batimastat (BB-94), Ilomastat (GM6001), Marimastat (BB2516), Thiols, Doxycycline, Squaric Acid, BB-1101, CGS-27023-A (MMI270B), COL-3 (metastat; CMT-3), AZD3342, Hydroxyureas, Hydrazines, Endogenous, Carbamoylphosphates, Beta Lactams, tetracycline and analogs and homologs of tetracycline, minocycline, 3-(4-phenoxyphenylsulfonyl)propylthiirane, pyrimidine-2,4-dione, BAY12-9566, prinomastat (AG-3340), N-{1S-[4-(4-Chlorophenyl)piperazine-1-sulfonylmethyl]-2-methylpropyl}-N-hydroxyformamide, RO 31-9790, 3-(4-PhenoxyphenylsulfonyDpropylthiirane, 1,6-bis[N'-(p-chlorophenyl)-N5-biguanido]hexane, trocade, sodium 1-(12-hydroxy)octadecanyl sulfate, minocycline (7-dimethylamino-6-dimethyl-6-deoxytetracycline), tetrapeptidylhydroxamic acid, N-[(2R)-2-(Carboxymethyl)-4-methylpentanoyl]-L-tryptophan-(S)-methyl-benzylamide, N-[(2R)-2-(Hydroxamidocarbonylmethyl)-4-methylpentanoyl]-L-tryptophan Methylamide, N-Hydroxy-1,3-di-(4-methoxybenzenesulphonyl)-5,5-dimethyl-[1,3]-piperazine-2-carboxamide, N-{1S-[4-(4-Chlorophenyl)piperazine-1-sulfonylmethyl]-2-methylpropyl}-N-hydroxyformamide, triaryl-oxy-aryloxy-pyrimidine-2,4,6-trione, 4r biarylbutyric acid, 5-biarylpentanoic acid, Fenbufen, peptide MMPIs, hydroxamic acid, tricyclic butyric acid, biphenyl butyric acid, heterocyclic substituted phenyl v butyric acid, sulfonamide, succinamide, FN-439 (p-aminobenzoyl-Gly-Pro-D-Leu-D-Ala-NHOH, MMP-Inh-1), sulfonated amino acid, MMP9 inhibitor I (CTK8G1150), ONO-4817, Ro 28-2653, SB-3CT, neutralizing anti-MMP antibody, and tacrolimus (FK506).

MMP inhibitors also include the family of tissue inhibitors of MMPs (TIMPs)). The term "TIMP," as used herein, means an endogenous tissue inhibitor of metalloproteinases, which is known to be involved in physiological/biological functions including the inhibition of active matrix metalloproteinases, regulation of pro-MMP activation, cell growth, and the modulation of angiogenesis. The human "TIMP family" contains four members, TIMP-1, TIMP-2, TIMP-3, and TIMP-4. The TIMP-1 protein is the most widely expressed and studied member of the TIMP family. Other members of the TIMP family include TIMP-2, TIMP-3 and TIMP-4. TIMP proteins not only share common structural features, including a series of conserved cysteine residues that form disulfide bonds essential for the native protein conformation (Brew et al., 2000), but they also have widely overlapping biological activities. The conserved N-terminal region of the TIMP proteins is necessary for functional inhibitory activities, while the divergent C-terminal regions are thought to modulate the selectivity of inhibition and binding efficiency of agents to the MMPs). However, apart from their ability to act as MMP inhibitors, the various TIMP family members may also exhibit additional biological activities, including the regulation of proliferation and apoptosis in addition to the modulation of angiogenic and inflammatory responses.

TIMP-1 has been found to inhibit most MMPs (except MMP-2 and -14), and preferentially inhibits MMP-8. TIMP-1 is produced and secreted in soluble form by a variety of cell types and is widely distributed throughout the body. It is an extensively glycosylated protein with a molecular mass of 28.5 kDa. TIMP-1 inhibits the active forms of MMPs, and complexes with the proform of MMP9. Like MMP9, TIMP-1 expression is sensitive to many factors. Increased synthesis of TIMP-1 is caused by a wide variety of reagents that include: TGF beta, EGF, PDGF, FGFb, PMA, alltransretinoic acid (RA), IL1 and IL11.

TIMP-2 is a 21 kDa glycoprotein that is expressed by a variety of cell types. It forms a non-covalent, stoichiometric complex with both latent and active MMPs. TIMP-2 shows a preference for inhibition of MMP-2.

TIMP-3 is typically bound to the ECM and inhibits the activity of MMP-1, -2, -3, -9, and 13. TIMP-3 shows 30% amino acid homology with TIMP-1 and 38% homology with TIMP-2. TIMP-3 has been shown to promote the detachment of transformed cells from ECM and to accelerate morphological changes associated with cell transformation.

Due to its high-affinity binding to the ECM, TIMP-3 is unique among the TIMPs. TIMP-3 has been shown to promote the detachment of transformed cells from the ECM and to accelerate the morphological changes associated with cell transformation. TIMP-3 contains a glucosaminoglycan (GAG) binding domain comprising six amino acids (Lys30, Lys26, Lys22, Lys42, Arg20, Lys45) that are thought to be responsible for an association with the cell surface. TIMP-3 is the only TIMP that normally inhibits TACE (TNF-α-converting enzyme), another metalloprotease that releases soluble TNF and is responsible for the processing of the IL-6 receptor to thus play a central part in the wound healing process.

TIMP-4 inhibits all known MMPs, and preferentially inhibits MMP-2 and -7. TIMP4 shows 37% amino acid identity with TIMP1 and 51% homology with TIMP2 and TIMP3. TIMP4 is secreted extracellularly, predominantly in heart and brain tissue and appears to function in a tissue specific fashion with respect to extracellular matrix (ECM) homeostasis.

The MMP inhibitor includes a substance that can permit or enhance adhesion of the MMP inhibitor to an outer surface of the staples (*e.g.* the staple legs or the staple crown). The substance is an absorbable polymer which can optionally be water soluble and/or ionically charged.

Suitable absorbable polymers that can permit or enhance adhesion of a MMP inhibitor to an outer surface may comprise synthetic and/or non-synthetic materials. Examples of non-synthetic materials include, but are not limited to, lyophilized polysaccharide, glycoprotein, bovine pericardium, collagen, gelatin, fibrin, fibrinogen, elastin, proteoglycan, keratin, albumin, hydroxyethyl cellulose, cellulose, oxidized cellulose, oxidized regenerated cellulose (ORC), hydroxypropyl cellulose, carboxyethyl cellulose, carboxymethylcellulose, chitan, chitosan, casein, alginate, and combinations thereof. Examples of synthetic absorbable materials include, but are not limited to, poly(lactic acid) (PLA), poly(L-lactic acid) (PLLA), polycaprolactone (PCL), polyglycolic acid (PGA), poly(trimethylene carbonate) (TMC), polyethylene terephthalate (PET), polyhydroxyalkanoate (PHA), a copolymer of glycolide and ε-caprolactone (PGCL), a copolymer of glycolide and -trimethylene carbonate, poly(glycerol sebacate) (PGS), poly(dioxanone) (PDS), polyesters, poly(orthoesters), polyoxaesters, polyetheresters, polycarbonates, polyamide esters, polyanhydrides, polysaccharides, poly(ester-amides), tyrosine-based polyarylates, polyamines, tyrosine-based polyiminocarbonates, tyrosine-based polycarbonates, poly(D,L-lactide-urethane), poly(hydroxybutyrate), poly(B-hydroxybutyrate), poly(ε-caprolactone), polyethyleneglycol (PEG), poly[bis(carboxylatophenoxy)phosphazene]poly(amino acids), pseudo-poly(amino acids), absorbable polyurethanes, poly(phosphazine), polyphosphazenes, polyalkyleneoxides, polyacrylamides, polyhydroxyethylmethylacrylate, polyvinylpyrrolidone, polyvinyl alcohols, poly(caprolactone), polyacrylic acid, polyacetate, polypropylene, aliphatic polyesters, glycerols, copoly(ether-esters), polyalkylene oxalates, polyamides, poly(iminocarbonates), polyalkylene oxalates, and combinations thereof. In various embodiments, the polyester is may be selected from the group consisting of polylactides, polyglycolides, trimethylene carbonates, polydioxanones, polycaprolactones, polybutesters, and combinations thereof.

In some embodiments, the MMP inhibitor can be absorbed into or encapsulated by the synthetic or non-synthetic absorbable polymer. Further, the polymer can have one or more attractive aspects associated with it to encourage adhesion of the MMP inhibitor to areas of the staple coated with it. For example, the absorbable polymer can be porous to allow liquid containing the MMP inhibitor to pool and collect in the pores where it is retained when the liquid dries. Additionally, one or both of the MMP inhibitor or the absorbable polymer can be formulated with an ionic charge, thereby permitting them to electrostatically attract. The absorbable polymer (for example, a polyurethane) can also be formulated to attach MMP inhibitor covalently as a pendent element to the polymer chain itself. Finally, the water solubility of the polymer itself can be manipulated to allow the polymer and the MMP inhibitor to co-mingle before water is removed from the construct leaving the drug and polymer blended.

In at least some implementations, an absorbable lubricant can be used to permit or enhance adhesion of a MMP inhibitor to an outer surface of a surgical stapler or component thereof (such as, the outer surface of a staple (*e.g.* the staple legs or the staple crown) or an adjunct material). Suitable absorbable lubricants can include, for example and without limitation, any of the common tablet water insoluble lubricants such as magnesium stearate, sodium stearate, calcium stearate, powdered stearic acid, talc, paraffin, cocoa butter, graphite, lycopodium or combinations thereof. Absorbable lubricants can be are fatty acid-derived, such as the stearates, for example, magnesium stearate, sodium stearate, calcium stearate and stearic acid.

### IMPLANTABLE ADJUNCTS

Various implantable adjuncts are provided for use in conjunction with surgical stapling instruments. "Adjuncts" are also referred to herein as "adjunct materials." The adjuncts can have a variety of configurations, and can be formed from various materials. In general, an adjunct can be formed from one or more of a film, a foam, an injection molded thermoplastic, a vacuum thermoformed material, a fibrous structure, and hybrids thereof. The adjunct can also include one or more biologically-derived materials and one or more drugs. Each of these materials is discussed in more detail below.

An adjunct can be formed from a foam, such as a closed-cell foam, an open-cell foam, or a sponge. An example of how such an adjunct can be fabricated is from animal derived collagen, such as porcine tendon, that can then be processed and lyophilized into a foam structure. Examples of various foam adjuncts are further described in previously mentioned U.S. Pat. No. 8,393,514 entitled "Selectively Orientable Implantable Fastener Cartridge" and filed September 30, 2010.

An adjunct can also be formed from a film formed from any suitable material or combination thereof discussed below. The film can include one or more layers, each of which can have different degradation rates. Furthermore, the film can have various regions formed therein, for example, reservoirs that can releasably retain therein one or more medicants (for example, at least one MMP inhibitor in a number of different forms. The reservoirs having at least one medicant disposed therein can be sealed using one or more different coating layers which can include absorbable or non-absorbable polymers. The film can be formed in various ways, for example, it can be an extruded or a compression molded film.

An adjunct can also be formed from injection molded thermoplastic or a vacuum thermoformed material. Examples of various molded adjuncts are further described in U.S. Pat. Pub. No. 2013/0221065 entitled "Fastener Cartridge Comprising A Releasably Attached Tissue Thickness Compensator" and filed February 8, 2013. The adjunct can also be a fiber-based lattice which can be a woven fabric, knitted fabric or non-woven fabric such as a melt-blown, needle-punched or thermal-constructed loose woven fabric. An adjunct can have multiple regions that can be formed from the same type of lattice or from different types of lattices that can together form the adjunct in a number of different ways. For example, the fibers can be woven, braided, knitted, or otherwise interconnected so as to form a regular or irregular structure. The fibers can be interconnected such that the resulting adjunct is relatively loose. Alternatively, the adjunct can include tightly interconnected fibers. The adjunct can be in a form of a sheet, tube, spiral, or any other structure that can include compliant portions and/or more rigid, reinforcement portions. The adjunct can be configured such that certain regions thereof can have more dense fibers while others have less dense fibers. The fiber density can vary in different directions along one or more dimensions of the adjunct, based on an intended application of the adjunct.

The adjunct can also be a hybrid construct, such as a laminate composite or melt-locked interconnected fiber. Examples of various hybrid construct adjuncts are further described in U.S. Pat. Pub. No. 2013/0146643 entitled "Adhesive Film Laminate" and filed February 8, 2013, and in U.S. Pat. No. 7,601,118 entitled "Minimally Invasive Medical Implant And Insertion Device And Method For Using The Same" and filed September 12, 2007.

The adjuncts can be formed from various materials. The materials can be used in various embodiments for different purposes. The materials can be selected in accordance with a desired therapy to be delivered to tissue so as to facilitate tissue in-growth. The materials described below can be used to form an adjunct in any desired combination.

The materials can include bioabsorbable and biocompatible polymers, including homopolymers and copolymers. Non-limiting examples of homopolymers and copolymers include p-dioxanone (PDO or PDS), polyglycolic acid (PGA), poly(lactic-co-glycolic acid) (PLGA), polycaprolactone (PCL), trimethylene carbonate (TMC), and polylactic acid (PLA), poly(glycolic acid-co-lactic acid) (PLA/PGA) (*e.g*., PLA/PGA materials used in Vicryl, Vicryl Rapide, PolySorb, and Biofix), polyurethanes (such as Elastane, Biospan, Tecoflex, Bionate, and Pellethane fibers), polyorthoesters, polyanhydrides (*e.g*., Gliadel and Biodel polymers), polyoxaesters, polyesteramides, and tyrosine-based polyesteramides. The copolymers can also include poly(lactic acid-co- polycaprolactone) (PLA/PCL), poly(L-lactic acid-co-polycaprolactone) (PLLA/PCL), poly(glycolic acid-co-trimethylene carbonate) (PGA/TMC) (*e.g.,* Maxon), Poly(glycolic acid-co-caprolactone) (PCL/PGA) (*e.g.,* Monocryl and Capgly), PDS/PGA/TMC (*e.g.,* Biosyn), PDS/PLA, PGA/PCL/TMC/PLA (*e.g.,* Caprosyn), and LPLA/DLPLA (*e.g*., Optima).

The adjuncts described herein can releasably retain therein at least one medicant that can be selected from a large number of different medicants. Medicants include, but are not limited to, any of the MMP inhibitors disclosed herein.

Other medicants for use with the adjuncts include, but are not limited to, for example, antimicrobial agents such as antibacterial and antibiotic agents, antifungal agents, antiviral agents, anti-inflammatory agents, growth factors, analgesics, anesthetics, tissue matrix degeneration inhibitors, anti-cancer agents, hemostatic agents, and other agents that elicit a biological response.

An adjunct can also include other active agents, such as active cell culture (*e.g.,* diced autologous tissue, agents used for stem cell therapy (*e.g*., Biosutures and Cellerix S.L.), hemostatic agents, and tissue healing agents. Non-limiting examples of hemostatic agents can include cellulose such as oxidized Regenerated Cellulose (ORC) (*e.g.,* Surgicel and Interceed), fibrin/thrombin (*e.g*., Thrombin-JMI, TachoSil, Tiseel, Floseal, Evicel, TachoComb, Vivostat, and Everest), autologous platelet plasma, gelatin (*e.g*., Gelfilm and Gelfoam), hyaluronic acid such as microfibers (*e.g*., yarns and textiles) or other structures based on hyaluronic acid, or hyaluronic acid-based hydrogels. The hemostatic agents can also include polymeric sealants such as, for example, bovine serum albumin and glutarldehyde, human serum albumin and polyethylene cross-linker, and ethylene glycol and trimethylene carbonate. The polymeric sealants can include FocalSeal surgical sealant developed by Focal Inc.

Non-limiting examples of antimicrobial agents include Ionic Silver, Aminoglycosides, Streptomycin, Polypeptides, Bacitracin, Triclosan, Tetracyclines, Doxycycline, Minocycline, Demeclocycline, Tetracycline, Oxytetracycline, Chloramphenicol, Nitrofurans, Furazolidone, Nitrofurantoin, Beta-lactams, Penicillins, Amoxicillin, Amoxicillin +, Clavulanic Acid, Azlocillin, Flucloxacillin, Ticarcillin, Piperacillin + tazobactam, Tazocin, Biopiper TZ, Zosyn, Carbapenems, Imipenem, Meropenem, Ertapenem, Doripenem, Biapenem, Panipenem/betamipron, Quinolones, Ciprofloxacin, Enoxacin, Gatifloxacin, Gemifloxacin, Levofloxacin, Lomefloxacin, Moxifloxacin, Nalidixic Acid, Norfloxacin, Sulfonamides, Mafenide, Sulfacetamide, Sulfadiazine, Silver Sulfadiazine, Sulfadimethoxine, Sulfamethizole, Sulfamethoxazole, Sulfasalazine, Sulfisoxazole, Bactrim, Prontosil, Ansamycins, Geldanamycin, Herbimycin, Fidaxomicin, Glycopeptides, Teicoplanin, Vancomycin, Telavancin, Dalbavancin, Oritavancin, Lincosamides, Clindamycin, Lincomycin, Lipopeptide, Daptomycin, Macrolides, Azithromycin, Clarithromycin, Erythromycin, Roxithromycin, Telithromycin, Spiramycin, Oxazolidinones, Linezolid, Aminoglycosides, Amikacin, Gentamicin, Kanamycin, Neomycin, Netilmicin, Tobramycin, Paromycin, Paromomycin, Cephalosporins, Ceftobiprole, Ceftolozane, Cefclidine, Flomoxef, Monobactams, Aztreonam, Colistin, and Polymyxin B.

Non-limiting examples of antifungal agents include Triclosan, Polyenes, Amphotericin B, Candicidin, Filipin, Hamycin, Natamycin, Nystatin, Rimocidin, Azoles, Imidazole, Triazole, Thiazole, Allylamines, Amorolfin, Butenafine, Naftifine, Terbinafine, Echinocandins, Anidulafungin, Caspofungin, Micafungin, Ciclopirox, and Benzoic Acid.

Non-limiting examples of antiviral agents include uncoating inhibitors such as, for example, Amantadine, Rimantadine, Pleconaril; reverse transcriptase inhibitors such as, for example, Acyclovir, Lamivudine, Antisenses, Fomivirsen, Morpholinos, Ribozymes, Rifampicin; and virucidals such as, for example, Cyanovirin-N, Griffithsin, Scytovirin, α-Lauroyl-L-arginine ethyl ester (LAE), and Ionic Silver.

Non-limiting examples of anti-inflammatory agents include non-steroidal anti-inflammatory agents (*e.g*., Salicylates, Aspirin, Diflunisal, Propionic Acid Derivatives, Ibuprofen, Naproxen, Fenoprofen, and Loxoprofen), acetic acid derivatives (*e.g*., Tolmetin, Sulindac, and Diclofenac), enolic acid derivatives (*e.g*., Piroxicam, Meloxicam, Droxicam, and Lornoxicam), anthranilic acid derivatives (*e.g*., Mefenamic Acid, Meclofenamic Acid, and Flufenamic Acid), selective COX-2 inhibitors (*e.g*., Celecoxib (Celebrex), Parecoxib, Rofecoxib (Vioxx), Sulfonanilides, Nimesulide, and Clonixin), immune selective anti-inflammatory derivatives, corticosteroids (*e.g*., Dexamethasone), and iNOS inhibitors.

Non-limiting examples of growth factors include those that are cell signaling molecules that stimulate cell growth, healing, remodeling, proliferation, and differentiation. Exemplary growth factors can be short-ranged (paracrine), long ranged (endocrine), or self-stimulating (autocrine). Further examples of the growth factors include growth hormones (*e.g*., a recombinant growth factor, Nutropin, Humatrope, Genotropin, Norditropin, Saizen, Omnitrope, and a biosynthetic growth factor), Epidermal Growth Factor (EGF) (*e.g*., inhibitors, Gefitinib, Erlotinib, Afatinib, and Cetuximab), heparin-binding EGF like growth factors (*e.g*., Epiregulin, Betacellulin, Amphiregulin, and Epigen), Transforming Growth Factor alpha (TGF-a), Neuroregulin 1-4, Fibroblast Growth Factors (FGFs) (*e.g.,* FGF1-2, FGF2, FGF11-14, FGF18, FGF15/19, FGF21, FGF23, FGF7 or Keratinocyte Growth Factor (KGF), FGF10 or KGF2, and Phenytoin), Insuline-like Growth Factors (IGFs) (*e.g.,* IGF-1, IGF-2, and Platelet Derived Growth Factor (PDGF)), Vascular Endothelial Growth Factors (VEGFs) (*e.g*., inhibitors, Bevacizumab, Ranibizumab, VEGF-A, VEGF-B, VEGF-C, VEGF-D and Becaplermin).

Additional non-limiting examples of the growth factors include cytokines, such as Granulocyte Macrophage Colony Stimulating Factors (GM-CSFs) (*e.g*., inhibitors that inhibit inflammatory responses, and GM-CSF that has been manufactured using recombinant DNA technology and via recombinant yeast-derived sources), Granulocyte Colony Stimulating Factors (G-CSFs) (*e.g*., Filgrastim, Lenograstim, and Neupogen), Tissue Growth Factor Beta (TGF-B), Leptin, and interleukins (ILs) (*e.g.,* IL-1a, IL-1b, Canakinumab, IL-2, Aldesleukin, Interking, Denileukin Diftitox, IL-3, IL-6, IL-8, IL-10, IL-11, and Oprelvekin). The non-limiting examples of the growth factors further include erythropoietin (*e.g*., Darbepoetin, Epocept, Dynepo, Epomax, NeoRecormon, Silapo, and Retacrit).

Non-limiting examples of analgesics include Narcotics, Opioids, Morphine, Codeine, Oxycodone, Hydrocodone, Buprenorphine, Tramadol, Non-Narcotics, Paracetamol, acetaminophen, NSAIDS, and Flupirtine.

Non-limiting examples of anesthetics include local anesthetics (*e.g*., Lidocaine, Benzocaine, and Ropivacaine) and general anesthetic.

Non-limiting examples of anti-cancer agents include monoclonial antibodies, bevacizumab (Avastin), cellular/chemoattractants, alkylating agents (*e.g*., Bifunctional, Cyclophosphamide, Mechlorethamine, Chlorambucil, Melphalan, Monofunctional, Nitrosoureas and Temozolomide), anthracyclines (*e.g*., Daunorubicin, Doxorubicin, Epirubicin, Idarubicin, Mitoxantrone, and Valrubicin), cytoskeletal disrupters (*e.g*., Paclitaxel and Docetaxel), epothilone agents that limit cell division by inhibiting microtubule function, inhibitor agents that block various enzymes needed for cell division or certain cell functions, histone deacetylase inhibitors (*e.g.,* Vorinostat and Romidepsin), topoisomerase I inhibitors (*e.g.,* Irinotecan and Topotecan), topoisomerase II inhibitors (*e.g*., Etoposide, Teniposide, and Tafluposide), kinase inhibitors (*e.g*., Bortezomib, Erlotinib, Gefitinib, Imatinib, Vemurafenib, and Vismodegib), nucleotide analogs (*e.g*., Azacitidine, Azathioprine, Capecitabine, Cytarabine, Doxifluridine, Fluorouracil, 5-FU, Adrucil, Carac, Efudix, Efudex, Fluoroplex, Gemcitabine, Hydroxyurea, Mercaptopurine, and Tioguanine), peptide antibiotic agents that cleave DNA and disrupt DNA unwinding/winding (*e.g*., Bleomycin and Actinomycin), platinum-based anti-neoplastic agents that cross link DNA which inhibits DNA repair and/or synthesis (*e.g*., Carboplatin, Cisplatin, Oxaliplatin, and Eloxatin), retinoids (*e.g*., Tretinoin, Alitretinoin, and Bexarotene), vinca alkaloids gents that inhibit mitosis and microtubule formation (*e.g*., Vinblastine, Vincristine, Vindesine, Vinorelbine), anti-ileus agents, pro-motility agents, immunosuppresants (*e.g*., Tacrolimus), blood aspect modifier agents (*e.g*., Vasodilator, Viagra, and Nifedipine), 3-hydroxy-3-methyl-glutaryl-CoA (HMG CoA) reductase inhibitors (*e.g*., Atorvastatin), and anti-angiogenesis agents.

Exemplary medicants also include agents that passively contribute to wound healing such as, for example, nutrients, oxygen expelling agents, amino acids, collageno synthetic agents, Glutamine, Insulin, Butyrate, and Dextran. Exemplary medicants also include anti-adhesion agents, non-limiting examples of which include Hyaluronic acid/ Carboxymethyl cellulose (seprafilm), Oxidized Regenerated Cellulose (Interceed), and Icodextrin 4% (Extraneal, Adept).

An adjunct in accordance with the described techniques can be associated with at least one medicant (*e.g.,* a MMP inhibitor) in a number of different ways, so as to provide a desired effect, such as on tissue in-growth, in a desired manner. The at least one medicant can be configured to be released from the adjunct in multiple spatial and temporal patterns to trigger a desired healing process at a treatment site. The medicant can be disposed within, bonded to, incorporated within, dispersed within, or otherwise associated with the adjunct. For example, the adjunct can have one or more regions releasably retaining therein one or more different medicants. The regions can be distinct reservoirs of various sizes and shapes and retaining medicants therein in various ways, or other distinct or continuous regions within the adjuncts. In some aspects, a specific configuration of the adjunct allows it to releasably retain therein a medicant or more than one different medicant.

Regardless of the way in which the medicant is disposed within the adjunct, an effective amount of the at least one medicant can be encapsulated within a vessel, such as a pellet which can be in the form of microcapsules, microbeads, or any other vessel. The vessels can be formed from a bioabsorbable polymer.

Targeted delivery and release of at least one medicant from an adjunct can be accomplished in a number of ways which depend on various factors. In general, the at least one medicant can be released from the adjunct material as a bolus dose such that the medicant is released substantially immediately upon delivery of the adjunct material to tissue. Alternatively, the at least one medicant can be released from the adjunct over a certain duration of time, which can be minutes, hours, days, or more. A rate of the timed release and an amount of the medicant being released can depend on various factors, such as a degradation rate of a region from which the medicant is being released, a degradation rate of one or more coatings or other structures used to retains the medicant within the adjuncts, environmental conditions at a treatment site, and various other factors. In some aspects, when the adjunct has more than one medicant disposed therein, a bolus dose release of a first medicant can regulate a release of a second medicant that commences release after the first medicant is released. The adjunct can include multiple medicants, each of which can affect the release of one or more other medicants in any suitable way.

Release of at least one medicant as a bolus dose or as a timed release can occur or begin either substantially immediately upon delivery of the adjunct material to tissue, or it can be delayed until a predetermined time. The delay can depend on a structure and properties of the adjunct or one or more of its regions.

### TEMPORARY INHIBITION OF WOUND HEALING

In further aspects, provided herein are methods for temporarily inhibiting wound healing at a surgical site immediately following surgical stapling of tissue by a surgical stapler. The method can include attaching an adjunct material to an end effector of a surgical stapler; engaging tissue between a cartridge assembly and an anvil of the end effector; and actuating the end effector to eject staples from the cartridge assembly into the tissue. The staples extend through the adjunct material to maintain the adjunct material at the surgical site. At least one of a portion of the plurality of staples (for example, the staple legs and/or crown) and/or the adjunct material can include a releasably effective amount of at least one MMP inhibitor. The term "effective amount" as used herein regarding the MMP inhibitor released in accordance with the devices and methods herein means an amount of MMP inhibitor which is sufficient to inhibit at least one MMP to such an extent as to result in increased strength in a tissue in the 1-4 days immediately following tissue stapling. Release of the MMP inhibitor at the stapling site in the tissue prevents MMP-mediated extracellular matrix degeneration during wound healing in the tissue in a predetermined manner. Further, release of the MMP inhibitor at the stapling site can inhibit inflammation at the wound site, thereby decreasing the infiltration of immune cells (such as macrophages, neutrophils, or T-cells) that would otherwise occur during the inflammatory stage of wound healing.

In some implementations, the MMP inhibitor can be configured to be released from the staples and/or adjunct material and into the surrounding tissue from two to three days following staple insertion. For example, the MMP inhibitor can be encapsulated in an absorbable polymer (such as any of the absorbable polymers disclosed herein) that begins to break down and release its contents starting at about 48 hours following staple insertion into the tissue and continues for about the next 24-36 hours (such as for about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or 36 hours).

Sufficient quantities of the MMP inhibitor can be released from the plurality of staples and/or adjunct material to inhibit at least five percent the proteolytic activity (such as inhibits any of 5%, 10%, 15%, 20%, 25%, 30%, 35%, 40%, 45%, 50%, 55%, 60%, 65%, 70%, 75%, 80%, 85%, 90%, 95%, or 100% of the proteolytic activity) of one or more MMP inhibitors that are expressed at the tissue stapling site immediately following staple insertion (such as those MMP inhibitors expressed within 1-4 days following stapling of tissue, for example, MMP1, MMP2, MMP8, and/or MMP9). In some embodiments, sufficient quantities of MMP inhibitor are released from the plurality of staples and/or adjunct material following staple insertion into tissue to ensure a concentration of at least about 0.1 nM to about 500µM of MMP inhibitor in the wound site surrounding the staples, such as any of about 0.1 nM, 0.2 nM, 0.3 nM, 0.4 nM, 0.5 nM, 0.6 nM, 0.7 nM, 0.8 nM, 0.9 nM, 1 nM, 2 nM, 3 nM, 4 nM, 5 nM, 6 nM, 7 nM, 8v, 9 nM, 10 nM, 11 nM, 12 nM, 13 nM, 14 nM, 15 nM, 1 nM 6 nM, 17 nM, 18 nM, 19 nM, 20 nM, 21 nM, 22 nM, 23 nM, 24 nM, 25 nM, 30 nM, 35 nM, 40 nM, 45 nM, 50 nM, 55 nM, 60 nM, 65 nM, 70 nM, 75 nM, 80 nM, 85 nM, 90 nM, 95 nM, 100 nM, 200 nM, 300 nM, 400 nM, 500 nM, 600 nM, 700 nM, 800 nM, 900 nM, 1µM, 2 µM, 3 µM, 4 µM, 5 µM, 10 µM, 15 µM, 20 µM, 25 µM, 30 uM, 35 µM, 40 uM, 45 µM, 50 uM, 55 µM, 60 uM, 65 µM, 70 µM, 75 µM, 80 µM, 85 µM, 90 µM, 95 µM, 100 µM, 125 µM, 150 µM, 175 µM, 200 µM, 225 µM, 250 µM, 275 µM, 300 µM, 325 µM, 350 µM, 375 µM, 400 µM, 425 µM, 450 µM, 475 µM, or 500 µM or more MMP inhibitor in the wound site, inclusive of numbers falling in between these values.

In further implementations, release of MMP inhibitors from the plurality of staples and/or adjunct material following staple insertion into tissue increases the strength of the tissue at the wound site due to increased production and maintenance of collagen fibers in the ECM immediately surrounding the staple insertion site. In some embodiments, the tissue surrounding staples and/or adjunct material releasably coated with at least one MMP inhibitor contains at least about 5%, 10%, 15%, 20%, 25%, 30%, 35%, 40%, 45%, 50%, 55%, 60%, 65%, 70%, 75%, 80%, 85%, 90%, 95%, or 100% or more increased collagen compared to tissue surrounding staples and/or adjunct material that are not releasably coated with at least one MMP inhibitor.

Immediately following staple insertion, the surrounding cells of a blood clot release inflammatory cytokines and growth factors which attract a variety of cells to the wound site. Specifically, after tissue injury, macrophages and neutrophils are stimulated to produce reactive oxygen species (ROS) and MMPs (for example MMP2, MMP8, and/or MMP9) to promote cell adhesion and ECM remodeling. For example, expression of MMP2 coincides with expression of laminin-332during enhanced keratinocyte migration in wound healing. Since both MMP2 and MMP9 can cleave the gamma-2 chain of laminin-322, they result in a promigratory and EGF-like fragment that binds EGF receptor to trigger cell migration of keratinocytes at the wound matrix.

During the inflammatory stage of wound healing, fibronectin and other ECM protein fragments can attract monocytes to the wound site from the bloodstream. Interactions at the wound site cause monocytes to undergo differentiation into macrophages which are stimulated by growth factors to ROS, MMPs, and multiple growth factors such as platelet-derived growth factor (PDGF), transforming growth factor-β (TGF-β), vascular endothelial growth factor (VEGF), and fibroblast growth factor (FGF). One factor that can stimulate macrophages is angiotensin II (AngII). The renin-angiotensin system (RAS) is a pathway to regulate angiotensin, a hormone peptide, to eventually produce the primary effector-AngII. This effector is present in macrophages, neutrophils, fibroblasts, and endothelial cells. With binding and stimulation of AngII, inflammatory cells such as macrophages generate ROS and MMPs that subsequently promote migration and proliferation of keratinocytes and other inflammation-promoting wound cells.

Accordingly, in some aspects, sufficient quantities of the MMP inhibitor are released from the plurality of staples and/or adjunct material to prevent or decrease (such as by any of 10%, 20%, 30%, 40%, 50%, 60%, 70%, 80%, 90%, or 100%) infiltration of one or more inflammation-promoting immune cells (such macrophages, neutrophils, or T-cells) into the wound surrounding the staple insertion site. Further, sufficient quantities of the MMP inhibitor are released from the plurality of staples and/or adjunct material to prevent or decrease (such as by any of 10%, 20%, 30%, 40%, 50%, 60%, 70%, 80%, 90%, or 100%) expression of one or more inflammation-promoting substances or enzymes (such as, for example, one or more of ROS, PDGF, TGF-β, VEGF, FGF, or AngII) at the wound site.

In a further embodiment, sufficient quantities of one or more MMP inhibitors are released from the staples and/or adjunct material to prevent or decrease collagen degradation in the ECM within 1 to 4 days (such as any of 1, 2, 3 or 4 days) following staple insertion. For example, use of MMP inhibitors in accordance with the surgical stapling apparatuses and methods disclosed herein can result in any of 5%, 10%, 15%, 20%, 25%, 30%, 35%, 40%, 45%, 50%, 55%, 60%, 65%, 70%, 75%, 80%, 85%, 90%, 95%, or 100% less collagen degradation in the ECM immediately surrounding a staple insertion site relative to the amount of ECM collagen degradation that would otherwise occur in the absence of one or more MMP inhibitors.

A person skilled in the art will appreciate that the present invention has application in conventional minimally-invasive and open surgical instrumentation as well application in robotic-assisted surgery.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

One skilled in the art will appreciate further features and advantages of the invention based on the above-described embodiments. Accordingly, the invention is not to be limited by what has been particularly shown and described, except as indicated by the appended claims.

## Claims

1. A staple cartridge assembly for use with a surgical stapler, comprising:
a cartridge body having a plurality of staple cavities, each staple cavity having one of a plurality of surgical staples disposed therein; and
wherein at least a portion of the plurality of staples include an effective amount of at least one matrix metalloproteinase (MMP) inhibitor being effective to prevent at least one of MMP-mediated extracellular matrix degeneration and inflammation during wound healing in the tissue in a predetermined manner,
**characterized in that** the at least one MMP inhibitor is disposed on at least a portion of the plurality of staples,
wherein the at least one MMP inhibitor comprises a substance that is configured to permit or enhance adhesion of the MMP inhibitor to an outer surface of the staples; and
wherein the substance is an absorbable polymer, and wherein the absorbable polymer is porous.

2. The assembly of claim 1, further comprising a biocompatible adjunct material releasably retained on the cartridge body and configured to be delivered to tissue by deployment of the staples in the cartridge body, wherein an effective amount of at least one MMP inhibitor is disposed within and releasable from the adjunct material.

3. The assembly of any one of the preceding claims, wherein the MMP inhibitor is configured to inhibit one or more of MMP1, MMP2, MMP3, MMP7, MMP8, MMP9, MMP10, MMP11, MMP12, MMP13, MMP14, MMP15, MMP16, MMP17, MMP19, MMP23A, MMP23B, MMP25, MMP25, MMP26, MMP27, and MMP28.

4. The assembly of claim 3, wherein the MMP inhibitor is configured to inhibit a collagenase, a gelatinase, a stromelysin, or a membrane-type MMP.

5. The assembly of any one of the preceding claims, wherein the MMP inhibitor is configured to inhibit MMP9.

6. The assembly of any one of the preceding claims, wherein the MMP inhibitor is a tissue inhibitor of metalloproteinase (TIMP).

7. The assembly of claim 6, wherein the TIMP is at least one of TIMP1, TIMP2, TIMP3 and TIMP4.

8. The assembly of any one of the preceding claims, wherein the MMP inhibitor is one or more of a hydroxymate, carboxylate, carbamoylphosphonate, hydroxyurea, hydrazine, β-lactam, squaric acid, nitrogenous ligand, thiol, and phosphinyl.

9. The assembly of any one of the preceding claims, wherein the MMP inhibitor is configured to prevent inflammation-causing cells from being attracted to the tissue following deployment of the staples.

## Patentansprüche

1. Klammermagazinanordnung zur Verwendung mit einem chirurgischen Klammerinstrument, umfassend:
einen Magazinkörper mit einer Vielzahl von Klammerhohlräumen, wobei jeder Klammerhohlraum darin angeordnet eine von einer Vielzahl chirurgischer Klammern aufweist; und
wobei mindestens ein Anteil der Vielzahl von Klammern eine effektive Menge von mindestens einem Matrixmetalloproteinase- (MMP)-Inhibitor einschließt, die effektiv ist, um mindestens eines von MMP-vermittelter extrazellulärer Matrixdegeneration und Entzündung während der Wundheilung in dem Gewebe in einer vorbestimmten Weise zu verhindern,
**dadurch gekennzeichnet, dass** der mindestens eine MMP-Inhibitor auf mindestens einem Anteil von der Vielzahl von Klammern angeordnet ist,
wobei der mindestens eine MMP-Inhibitor eine Substanz umfasst, die ausgestaltet ist, um Adhäsion des MMP-Inhibitors an einer Außenfläche der Klammern zuzulassen oder zu verstärken; und
wobei die Substanz ein absorbierbares Polymer ist, und wobei das absorbierbare Polymer porös ist.

2. Anordnung nach Anspruch 1, des Weiteren umfassend ein biokompatibles Hilfsmaterial, das freigebbar auf dem Magazinkörper gehalten wird und ausgestaltet ist, um durch Ausbringung der Klammern in dem Magazinkörper an Gewebe abgegeben zu werden, wobei eine effektive Menge von mindestens einem MMP-Inhibitor innerhalb des Hilfsmaterials und freigebbar aus diesem angeordnet ist.

3. Anordnung nach einem der vorhergehenden Ansprüche, wobei der MMP-Inhibitor ausgestaltet ist, um ein oder mehrere von MMP1, MMP2, MMP3, MMP7, MMP8, MMP9, MMP10, MMP11, MMP12, MMP13, MMP14, MMP15, MMP16, MMP17, MMP19, MMP23A, MMP23B, MMP25, MMP25, MMP26, MMP27 und MMP28 zu inhibieren.

4. Anordnung nach Anspruch 3, wobei der MMP-Inhibitor ausgestaltet ist, um eine Kollagenase, eine Gelatinase, ein Stromelysin oder ein MMP vom Membrantyp zu inhibieren.

5. Anordnung nach einem der vorhergehenden Ansprüche, wobei der MMP-Inhibitor ausgestaltet ist, um MMP9 zu inhibieren.

6. Anordnung nach einem der vorhergehenden Ansprüche, wobei der MMP-Inhibitor ein Gewebeinhibitor von Metalloproteinase (TIMP) ist.

7. Anordnung nach Anspruch 6, wobei der TIMP mindestens einer von TIMP1, TIMP2, TIMP3 und TIMP4 ist.

8. Anordnung nach einem der vorhergehenden Ansprüche, wobei der MMP-Inhibitor ein oder mehrere von einem Hydroxymat, Carboxylat, Carbamoylphosphonat, Hydroxyharnstoff, Hydrazin, β-Lactam, Quadratsäure, stickstoffhaltigem Liganden, Thiol und Phosphinyl ist.

9. Anordnung nach einem der vorhergehenden Ansprüche, wobei der MMP-Inhibitor ausgestaltet ist, um zu verhindern, dass nach Ausbringen der Klammern Entzündung verursachende Zellen zu dem Gewebe gelockt werden.

## Revendications

1. Ensemble cartouche d'agrafes destiné à une utilisation avec une agrafeuse chirurgicale, comprenant :
un corps de cartouche possédant une pluralité de cavités pour agrafes, chaque cavité pour agrafe ayant une agrafe d'une pluralité d'agrafes chirurgicales disposée en son sein ; et
dans lequel au moins une partie de la pluralité d'agrafes comprend une quantité efficace d'au moins un inhibiteur de métalloprotéinase matricielle (MMP) étant efficace pour prévenir une dégénérescence et/ou une inflammation de la matrice extracellulaire à médiation MMP pendant la cicatrisation d'une plaie dans le tissu d'une manière prédéfinie,
**caractérisé en ce que** l'au moins un inhibiteur de MMP est disposé sur au moins une partie de la pluralité d'agrafes, dans lequel l'au moins un inhibiteur de MMP comprend une substance qui est configurée pour permettre ou améliorer une adhérence de l'inhibiteur de MMP à une surface externe des agrafes ; et
dans lequel la substance est un polymère résorbable, et dans lequel le polymère résorbable est poreux.

2. Ensemble selon la revendication 1, comprenant en outre un matériau auxiliaire biocompatible retenu de manière libérable sur le corps de cartouche et conçu pour être posé sur un tissu par le déploiement des agrafes dans le corps de cartouche, dans lequel une quantité efficace d'au moins un inhibiteur de MMP est disposée à l'intérieur du matériau auxiliaire et libérable depuis celui-ci.

3. Ensemble selon l'une quelconque des revendications précédentes, dans lequel l'inhibiteur de MMP est configuré pour inhiber un ou plusieurs parmi MMP1, MMP2, MMP3, MMP7, MMP8, MMP9, MMP10, MMP11, MMP12, MMP13, MMP14, MMP15, MMP16, MMP17, MMP19, MMP23A, MMP23B, MMP25, MMP25, MMP26, MMP27 et MMP28.

4. Ensemble selon la revendication 3, dans lequel l'inhibiteur de MMP est configuré pour inhiber une collagénase, une gélatinase, une stromélysine ou un MMP de type membrane.

5. Ensemble selon l'une quelconque des revendications précédentes, dans lequel l'inhibiteur de MMP est configuré pour inhiber MMP9.

6. Ensemble selon l'une quelconque des revendications précédentes, dans lequel l'inhibiteur de MMP est un inhibiteur tissulaire de métalloprotéinase (TIMP).

7. Ensemble selon la revendication 6, dans lequel le TIMP est TIMP1 et/ou TIMP2 et/ou TIMP3 et/ou TIMP4.

8. Ensemble selon l'une quelconque des revendications précédentes, dans lequel l'inhibiteur de MMP est un ou plusieurs parmi un hydroxymate, un carboxylate, un carbamoylphosphonate, de l'hydroxyurée, de l'hydrazine, du β-lactame, de l'acide squarique, un ligand azoté, du thiol et du phosphinyle.

9. Ensemble selon l'une quelconque des revendications précédentes, dans lequel l'inhibiteur de MMP est conçu pour empêcher que des cellules provoquant une inflammation soient attirées vers le tissu après le déploiement des agrafes.
